# EUROPEAN PATENT APPLICATION

(11) **EP 3 502 258 A1**
(43) Date of publication of application: **26.06.2019**
(21) Application number: 17209585.3
(22) Date of filing: 21.12.2017
(51) Int. Cl.: C12N 15/67

(54) **CLICK-MODIFIED IN VITRO TRANSCRIBED MRNA FOR GENE EXPRESSION**

(71) Applicant: baseclick GmbH, 82061 Neuried (DE)
(72) Inventor: Frischmuth, Thomas, 12247 Berlin (DE); Serdjukow, Sascha, 83088 Kiefersfelden (DE); Graf, Birgit, 80469 München (DE); Croce, Stefano, 81669 München (DE)
(74) Representative: Weickmann & Weickmann PartmbB

(57) **Abstract**

The present invention relates to alkyne- and/or azide-modified mRNA, processes for producing such modified mRNA, cells which are transfected to include the modified mRNA, pharmaceutical compositions containing the modified mRNA or cells including the modified mRNA, and to uses of such mRNA, cells or pharmaceutical compositions in mRNA based therapeutic and/or prophylactic applications.

SEQUENCE LISTING

<110>  baseclick GmbH

<120>  Click-modified in vitro transcribed mRNA for gene expression

<130>  64932P EP

<160>  2

<170>  PatentIn version 3.5

<210>  1
<211>  31
<212>  RNA
<213>  Artificial Sequence

<220>
<223>  RNA oligonucleotide

<400>  1
cuagugcagu acauguaauc gaccagauca a                                    31


<210>  2
<211>  1239
<212>  DNA
<213>  Artificial Sequence

<220>
<223>  partial sequence of pSTI-A120-luc+rbs (from T7 promoter to
       poly(A))

<400>  2
taatacgact cactataggg cgaactagta agcaaggagg cgtgcagatg gtgagcaagg      60

gcgaggagct gttcaccggg gtggtgccca tcctggtcga gctggacggc gacgtaaacg     120

gccacaagtt cagcgtgtcc ggcgagggcg agggcgatgc cacctacggc aagctgaccc     180

tgaagttcat ctgcaccacc ggcaagctgc ccgtgccctg gcccaccctc gtgaccaccc     240

tgacctacgg cgtgcagtgc ttcagccgct accccgacca catgaagcag cacgacttct     300

tcaagtccgc catgcccgaa ggctacgtcc aggagcgcac catcttcttc aaggacgacg     360

gcaactacaa gacccgcgcc gaggtgaagt tcgagggcga caccctggtg aaccgcatcg     420

agctgaaggg catcgacttc aaggaggacg gcaacatcct ggggcacaag ctggagtaca     480

actacaacag ccacaacgtc tatatcatgg ccgacaagca gaagaacggc atcaaggtga     540

acttcaagat ccgccacaac atcgaggacg gcagcgtgca gctcgccgac cactaccagc     600

agaacacccc catcggcgac ggccccgtgc tgctgcccga caaccactac ctgagcaccc     660

agtccgccct gagcaaagac cccaacgaga agcgcgatca catggtcctg ctggagttcg     720

tgaccgccgc cgggatcact ctcggcatgg acgagctgta caagtccggc cggactcaga     780

tctcgagctc aagcttcgaa ttgatccaga tcttaagtaa gtaagctcga gagctcgctt     840

tcttgctgtc caatttctat taaaggttcc tttgttccct aagtccaact actaaactgg     900

gggatattat gaagggcctt gagcatctgg attctgccta ataaaaaaca tttattttca 960

ttgctgcgtc gagagctcgc tttcttgctg tccaatttct attaaaggtt cctttgttcc 1020

ctaagtccaa ctactaaact gggggatatt atgaagggcc ttgagcatct ggattctgcc 1080

taataaaaaa catttatttt cattgctgcg tcgagagcta aaaaaaaaaa aaaaaaaaaa 1140

aaaaaaaaaa aaaaaaaaaa aaaaaaaaaa aaaaaaaaaa aaaaaaaaaa aaaaaaaaaa 1200

aaaaaaaaaa aaaaaaaaaa aaaaaaaaaa aaaaaaaaa 1239

## Description

The present invention relates to alkyne- and/or azide-modified mRNA, processes for producing such modified mRNA, cells which are transfected to include the modified mRNA, pharmaceutical compositions containing the modified mRNA or cells including the modified mRNA, and to uses of such mRNA, cells or pharmaceutical compositions in mRNA based therapeutic and/or prophylactic applications.

### Background of the invention

Messenger RNA (mRNA) is the template molecule that is transcribed from cellular DNA and is translated into an amino acid sequence, i.e. a protein, at ribosomes in the cells of an organism. In order to control the expression level of the encoded proteins, mRNAs possess untranslated regions (UTRs) flanking the actual open reading frame (ORF) which contains the genetic information encoding the amino acid sequence. Such UTRs, termed the 5'-UTR and the 3'-UTR, respectively, are sections of the mRNA located before the start codon and after the stop codon. Further, mRNA contains a poly(A) tail region which is a long sequence of adenine nucleotides which promotes export of mRNA from the nucleus, translation and to some extent protects the mRNA from degradation.

Due to its chemical and biochemical properties, mRNA usually is degraded within a few minutes inside of cells, thus expression of a specific protein usually is a transient process. Moreover, the polyanionic mRNA molecule is not well suited to cross a cell membrane which renders external delivery of mRNA extremely difficult.

Despite these challenges associated with mRNA, scientific and technological advances of the recent years have made mRNA a promising candidate for a novel class of drugs. Sahin U. et al., Nat.Publ.Gr. 13, 759-780 (2014) provide an overview on mRNA-based therapeutics and drug development. mRNA is for example used to trigger *in vivo* production of proteins like antibodies and enzymes, or to stimulate an immune response, e.g. by expressing specific epitopes or via innate immune response towards structural mRNA parts. For example, RIG-1 binds 5'-triphosphate ends of RNA and triggers a signal cascade which results in activation of transcription factors and release of cytokines as parts of an antiviral response. Application of mRNA for stimulation of an immune response can be used in novel approaches to treat cancer, AIDS and to generate vaccines against almost any disease (cf. Pardi, N. et al., Nat.Publ.Gr. 543, 248-251 (2017) and Schlake T. et al., RNA Biol. 9, 1319-30 (2012)). Key to these exciting developments is the robust *in vitro* production of stabilized mRNA with improved translation efficiency and its delivery into cells using special transfection formulations.

mRNA stability and translation efficiency depend on several factors. Especially the untranslated regions at either ends of the mRNA play a crucial role. In eukaryotic protein expression, a cap structure at the 5'-end and the poly(A) tail at the 3'-end both increase mRNA stability and enhance protein expression. In addition, the 5'-UTR contains a ribosome binding site necessary for translation and the 3'-UTR contains RNA sequences that adopt secondary structures which improve stability and influence translation. Moreover, modified natural, e.g. *N*1-methylpseudouridine, and artificial nucleotides can be incorporated to improve mRNA stability and enhance translation of the mRNA (Svitkin Y.V. et al., Nucleic Acids Research, Vol. 45, No. 10, 6023-6036 (2017)).

Delivery of mRNAs into cells can be achieved by providing mixtures containing lipids for fusion with the cellular membrane and cations to neutralize the negative charge of the oligonucleotide backbone. Special formulations have been created to optimize mRNA delivery and to confer sufficient *in vivo* stability for clinical trials. Most of the mRNA formulations which are applied intravenously are taken up by and are expressed inside liver cells. This is due to the fact that the liver plays a major role in fatty acid metabolism and a high lipid content of the mRNA formulations therefore displays an organ-specific targeting effect. In most cases the liver is, however, not the desired target and therefore efforts are being made to modify lipid formulations to target organs, which are involved in an immune response, like e.g. the spleen (Kranz L.M. et al., Nature 534, 396-401 (2016)). Alternatively, cells of the immune system (e.g. lymphocytes) can be isolated from a patients' blood and mRNA application is performed *ex vivo* to allow targeting. Most recently, tissue-specific targeting of mRNA using antibody fragment modified lipid formulations has been disclosed (Moffett H.F. et al., Nat. Commun. 8, 389 (2017)).

Despite the recent advances and developments regarding the therapeutic applicability of mRNA either directly or indirectly, i.e. via *ex vivo* transfection of cells and returning such transfected cells to a patient, further improving the stability of mRNAs and developing new options in the context of their use as therapeutics or drugs are objects of ongoing research. Moreover, it is desirable to provide methods that allow for a streamlined and efficient production of therapeutic mRNA. Finally, there is still a need for advanced targeted delivery of mRNA for protein substitution and gene replacement therapies, especially in the context of the treatment of inherited diseases, and for further options for exploiting the immune stimulatory effect of mRNAs in e.g. cancer therapy.

### Summary of the invention

The present invention is directed to providing solutions to the above mentioned objects and relates to a new kind of mRNA modification. Such modification not only allows to stabilize mRNAs of interest for *ex vivo* application, for administration to a patient, animal or plant, but also to easily attach detectable labels or functional groups which e.g. allows to monitor delivery of mRNA to cells and to a specific cell or tissue type and deliver the modified mRNA to such cells or tissues.

In a first aspect, the present invention relates to modified mRNA which comprises a 5'-cap structure, a 5'-untranslated region (5'-UTR), an open reading frame region (ORF), a 3'-untranslated region (3'-UTR) and a poly(A) tail region, wherein the mRNA contains at least one of an alkyne- or azide modification in the nucleotides within at least one of the ORF, the 5'-UTR, the 3'-UTR and the poly(A) tail region. In especially preferred embodiments of this first aspect of the present invention, the modified mRNA contains one or more of a detectable label and / or a functional molecule introduced via a click reaction of the modified mRNA with a correspondingly modified alkyne- or azide-containing detectable label or functional molecule.

In a second aspect, the invention relates to a process for producing the modified mRNA according to the present invention, wherein such process comprises *in vitro* transcription of mRNA from a DNA template in the presence of an RNA polymerase and a nucleotide mixture containing the nucleotides required for RNA transcription, wherein at least a part of the nucleotides in the nucleotide mixture is modified to contain an alkyne- or azide-modification at the nucleotide.

A third aspect of the present invention relates to an enzymatic method for the preparation of a site-specifically modified mRNA of the invention which contains an alkyne- or azide-modification in a defined region of the mRNA, e.g. the poly(A) tail region only. Such process comprises performing a poly(A) polymerase addition reaction on an mRNA in the presence of adenosine triphosphate (ATP), wherein the ATP is at least partly alkyne- or azide-modified at the nucleotide.

In especially preferred embodiments of the second and third aspects of the invention, one or more of correspondingly alkyne- or azide-modified detectable labels and / or functional molecules are added under conditions to perform a click reaction to produce a modified mRNA comprising such detectable label(s) or functional molecule(s).

A fourth aspect of the present invention relates to a cell preparation, especially a preparation of cells of the immune system, which contains the modified mRNA of the present invention and is obtained by *ex vivo* transfection.

A fifth aspect of the present invention relates to pharmaceutical compositions which comprise as an active agent a modified mRNA of the present invention or a cell preparation which was obtained by *ex vivo* transfection to include such mRNA.

A still further and sixth aspect of the present invention is the use of a modified mRNA according to the invention, of a cell preparation including such mRNA or of a pharmaceutical composition of the invention in mRNA-based therapeutic and/or prophylactic applications

A seventh aspect of the invention are diagnostic compositions for *in vitro* or *in vivo* screening for the presence, delivery and/or distribution of the inventive mRNA in cells, tissues or organs, such compositions comprising a modified mRNA of the present invention containing or afterwards being modified with a detectable label, preferable a fluorophore or a radionuclide.

An eighth aspect of the invention relates to a kit of parts for preparing and/or delivering a modified mRNA of the present invention. In especially preferred embodiments, such kit also contains one or more of correspondingly alkyne- or azide-modified detectable labels or functional molecules to obtain modified mRNAs containing such detectable labels or functional molecules.

### Detailed description of the invention and preferred embodiments

The present invention employs so-called "click chemistry" or elements thereof and applies this technique to modify mRNA molecules to impart improved stability and/or to provide for use of such modified mRNA molecules in the context of *inter alia* mRNA based therapy and mRNA vaccine technologies.

Click chemistry is a concept which was defined in 2001/2002 by the groups of Sharpless and Meldal (Sharpless, K.B. et al., Angew. Chem 2002, 114, 2708; Angew. Chem. Int. Ed. 2002, 41, 2596; Meldal, M. et al., J. Org. Chem. 2002, 67, 3057). Since then, especially the copper catalyzed reaction of azides with alkynes to give 1,2,3-triazoles, a variation of the 1,3-dipolar Huisgen cycloaddition (R. Huisgen, 1,3-Dipolar Cycloaddition Chemistry (Ed.: A. Padwa), Wiley, New York, 1984), has become a very widely used method to perform a click reaction. As a result of its mild conditions and high efficiency, this reaction has found a myriad of applications in biology and material sciences, such as e. g. DNA labeling for various purposes (Gramlich, P.M.A. et al., Angew. Chem. Int. Ed. 2008, 47, 8350).

In addition to the copper-catalyzed click-reaction, also copper-free, bio-orthogonal methods have been developed and such methods can also be employed in the context of the present invention. E.g., strain-promoted azide-alkyne cycloaddition (SPAAC,) (I.S. Marks et.al., Bioconjug Chem. 2011 22(7): 1259-1263) can be used either alone or in combination with copper-catalyzed click chemistry (CuAAC) in the context of the present invention. Especially in cases in which it is desirable to perform a labelling reaction *in vivo* in cell culture or in a living organism, performing such reaction using SPAAC is preferable since the method does not require the use of toxic substances or external catalysts.

Click chemistry facilitates attaching reporter molecules or labels to biomolecules of interest and is a very powerful tool for identifying, locating, and characterizing such biomolecules. The method for example enables inclusion and attachment of fluorescent probes for spectrometric quantification, or of anchor molecules to allow for separation and purification of the target biomolecules. Up to date, many applications have been developed in which click chemistry is used as an underlying principle. Next-generation sequencing is one of such applications which benefits from this technique where formation of so-called "backbone mimics", i.e. non-natural alternatives for the phosphodiester bond, which can be generated by copper-catalyzed azide alkyne cycloaddition (CuACC), is used to ligate e.g. DNA fragments and adapter sequences. Despite the presence of a triazole ring instead of a phosphodiester bond, such backbone mimics are acceptable substrates for polymerase driven DNA or RNA preparation methods like PCR or reverse transcription. Detection of cell proliferation is a further field of application for click-chemistry. The methods that are normally applied include adding either BrdU or radioactive nucleoside analogs to cells during replication and detecting their incorporation into DNA. Methods involving radioactivity, however, are rather slow and not suitable for rapid high-throughput studies and are also inconvenient because of the radioactivity involved. Detecting BrdU requires an anti-BrdU antibody and applying denaturing conditions resulting in degradation of the structure of the specimen. The development of EdU-click assays has overcome such limitations by including 5-ethynyl-2'-deoxyuridine, a thymidine analog, in the DNA replication reaction. The detection via click chemistry instead of an antibody is selective, straight forward, bioorthogonal and does not require DNA denaturation for the detection of the incorporated nucleoside.

Within the context of the present invention it was discovered that it is possible to introduce alkyne- and/or azide-modified nucleotides during *in vitro* transcription of mRNA to result in a correspondingly modified mRNA, wherein the modification can be included in only some or all elements contained in the mRNA. The presence of such modification on the one hand stabilizes the mRNA and on the other hand provides specific anchor sites for post-enzymatic labeling or for attachment of tissue- or cell-specific ligands or targeting molecules via click chemistry. Thus, the present invention not only enables detection of the presence and the location of mRNA after transfection or application, but also provides new options for targeted delivery of mRNAs to specific organs or cell types in the context of therapeutic applications. A correspondingly modified mRNA is a first subject matter of the present invention.

Depending on which type of nucleotide or nucleotides are included in alkyne- or azide-modified form during *in vitro* transcription, the resulting modified mRNA can contain modifications not only in the 5'- and 3'-UTRs and the ORF, but also in the poly(A) tail region. Including e.g. one or more of modified CTP, GTP and UTP leads to a modification within the UTRs and the ORF, while additionally including modified ATP results in a modification also of the poly(A) tail region. Including only alkyne- and / or azide-modified ATP during the transcription leads to modifications in the UTRs, the ORF and the poly(A) tail region.

No severe negative effects caused by the presence of alkyne- or azide-modified nucleotides within the mRNA of the invention have been observed. Depending on the amount of modified nucleotides included in the reaction, the *in vitro* transcription efficiency can be as effective as in cases where only non-modified nucleotides are present in the reaction mixture, or slightly decreased Furthermore, the modification of the mRNA does not seem to impair translation of mRNA during protein production at the ribosomes.. Depending on the circumstances, the amounts of modified nucleotides to be included in the *in vitro* transcription reaction can be adjusted to either provide maximum mRNA yield or maximum modification. For instance, when a dye is attached to the mRNA as a detectable label, it might be desirable to include an adequately high amount thereof to ensure and facilitate detection, whereas in order to target specific cell receptors, it might be sufficient to include only one or a few respective ligand molecules to achieve the desired effect.

It is to be expected that the stabilizing effect of the inventive modification is most pronounced if such modification is distributed over the complete mRNA molecule. Subsequent attachment of detectable labels and/or functional molecules via click chemistry will then occur also uniformly over the whole mRNA molecule.

However, in some cases it may be important to restrict the inclusion of labels or functional molecules to a part of the mRNA molecule which is not involved in subsequent translation of mRNA during protein expression. For such purpose, it can be desirable to include modified nucleotides in the poly(A) tail region only whereby it can be ensured that ribosomal activity is not impaired by the presence of especially longer or bulkier labels or functional molecules.

The present invention therefore also provides a modified mRNA containing alkyne- or azide-modification in the poly(A) tail region only. Instead of including alkyne- or azide modified nucleotides during *in vitro* transcription of a DNA template, a modification in only the poly(A) tail region can be achieved for any desired mRNA by performing an addition reaction in the presence of poly(A) polymerase and alkyne- or azide-modified ATP.

By controlling the amount and type of alkyne- or azide-modification in the modified mRNA of the invention it is possible to conveniently and easily adapt the resulting mRNA to impart stabilization and options for post-enzymatic attachment of molecules of interest as required and viable with regard to any intended application.

Within the context of the present invention the azide- or alkyne-modification can be included at the nucleobase or at the 2'-position of the ribose unit of the respective nucleotide. In very special cases, inclusion of a nucleotide containing the modification at the 3'-position is possible. In such case, the enzymatic poly(A) addition reaction is terminated upon inclusion of one modified nucleotide.

The modified nucleotide included in the mRNA of the present invention can be derived from a natural nucleotide and especially one of the standard nucleotides with adenine, cytosine, guanine or uracil bases, or it can be a modification of another naturally occurring nucleotide (e.g. pseudouridine derivative) or even a non-naturally occurring molecule which does not negatively affect transcription and/or translation and the function of the resulting modified mRNA. Preferably the modified nucleotide is derived from a natural nucleotide or a naturally occurring nucleotide within mRNA.

The alkyne-modified nucleotide preferably is an ethynyl-modified nucleotide, more preferably 5-ethynyluridine phosphate or 7-ethynyl-7-deazaadenine phosphate. While it is in principle also possible to employ higher alkyne-modified nucleotides, possible negative effects on the transcription or poly(A) polymerase reaction efficiency as well as on a further translation of the mRNA into a protein will have to be considered when selecting suitable alkyne molecules. As an azide-modified nucleotide, preferably 5-(3-azidopropyl)-uridine phosphate or 8-azidoadenine phosphate are preferably considered for inclusion in the inventive mRNA.

In principle, all nucleotides of the at least one type of modified nucleotide can be alkyne-or azide-modified, or alternatively, only a part of such nucleotides is present in modified form. In a preferred embodiment of the present invention and depending on the desired modification and modification rate, ratios of modified versus non-modified forms of the various nucleotides can range from 1:100 to 10:1, preferably 1:10 to 10:1, further preferably 1:4 to 4:1, and also preferably 1:2 to 2:1. Preferably, a 1:1, 1:4 or 1:10 combination of modified to non-modified nucleotide is included in the mRNA of the invention.

As mentioned above, the presence of alkyne-or azide-modified nucleotides or nucleobases in the modified mRNAs of the present invention confers a stabilizing effect. On the one hand, the attack of endoribonucleases is restricted to some extent by an internal modification. Extension of the poly(A) tail region during poly(A) polymerase based addition of modified ATPs at the 3'-end leads to a further stabilizing effect. Attack on and degradation of mRNA molecules by exoribonucleases occurs at the two ends of RNA. The mRNA according to the invention contains a cap at the 5'-end which provides protection from degradation at that side. Including additional modified adenosine nucleotides at the 3'-end imparts further protection as the attack of exoribonucleases in 3'→5' direction is impeded and degradation reaching the core mRNA, especially the ORF is delayed.

In a preferred embodiment of the present invention, detectable labels and / or functional molecules can be introduced to the modified mRNA via click reaction with a correspondingly modified alkyne- or azide-containing label or functional molecule. The reaction of an alkyne-modified nucleotide within the modified mRNA and an azide containing label or functional molecule or the reaction of an azide-modified nucleotide within the modified mRNA of the invention and an alkyne containing label or functional molecule under appropriate conditions to conduct the click reaction leads to formation of the 5-membered heterocyclic 1,2,3-triazole moiety which forms the link between mRNA and label or functional molecule. According to the present invention, the term alkyne-containing label or functional molecule also encompasses C-C triple bond-containing ring systems like cyclooctynes which are considered especially in the context of SPAAC reactions and *in vivo* labelling.

The type and size of labels and functional molecules are not particularly restricted and, again, are determined by the intended use. Preferred examples for the detectable label include color imparting or a fluorescence imparting labels, e.g. fluorescein derivatives like FITC, Alexa Fluor dyes or DyLight Fluor dyes, cyanine dyes like Cy5 and Cy3 or rhodamine dyes like Texas Red and 5-TAMRA, or any other fluorescent dye. Even non-colored small molecules can be used (e.g. biotin), when they are substrates for an enzyme or a binding protein-enzyme conjugate (e.g. antibody enzyme conjugates) and can produce a colored or luminescent product through an enzymatic reaction cascade and a further substrate. Also radionuclides can be included as detectable labels, e.g. preferably positron emitting radionuclides which can be detected using positron emission tomography scan. Depending on the intended use, also heavy isotopes like C¹³ or P³³ can be considered as a detectable label for the present invention.

Functional molecules to be included in the modified mRNA via a click reaction are not restricted and are preferably cell- or tissue-specific ligands that mediate targeted uptake of the mRNA into specific tissues or cells including cancer cells. Such cell- or tissue-specific targeting can be achieved for example by using specific antibodies or antibody fragments, peptides, sugar moieties or fatty acid moieties as the cell- or tissue-specific ligands. Respective substances have been described for a large number of targeting applications and are available to the skilled person. Some preferred and exemplary targeting molecules are antibodies or antibody fragments or receptor ligands which target cell specific receptors like e.g. the epidermal growth factor receptor, folate which targets the folate receptor, apolipoproteins which target endogenous low density lipoprotein receptors or arachidonic acid which targets the endogenous cannabinoid receptors. Also the amino acid sequence RGD or similar sequences have been found to mediate cell adhesion and can also be considered as preferred ligands within the context of the present invention.

The presence of functional molecules attached to the mRNA can further increase mRNA stability against nuclease degradation and it has been shown that partial as well as full replacement of at least one of the natural nucleotides within the mRNA for an alkyne- or azide-modified analogue and even attachment of functional molecules thereto does not hamper translation of the mRNA molecule.

In addition to including either alkyne-modified or azide-modified nucleotides, it is also possible that an inventive modified mRNA contains at least one nucleotide in at least partially alkyne-modified form and at least one, preferably other, nucleotide in at least partially azide-modified form. A further option is an mRNA including at least one type of nucleotide in alkyne- as well as azide-modified form. Such mRNA contains two different anchor modifications to which different labels or functional molecules can be attached in a downstream post-enzymatic click reaction. For example, but without limiting to such specific embodiment, an alkyne-modified cell specific targeting group as well as an azide-modified detectable label can then be attached resulting in another preferred embodiment of a modified mRNA according to the present invention.

It is also possible and preferred within the context of the invention to provide a modified mRNA containing at least an azide-modified nucleotide and an alkyne-modified nucleotide, wherein e.g. at the azide-modified nucleotide a detectable label or a functional molecule has been attached via a SPAAC reaction *in vitro,* whereas the alkyne-modified nucleotide is available for a downstream *in vitro* labelling reaction via a CuAAC reaction.

It is for example also conceivable for the inventive modified mRNA to contain one kind of modification in the UTRs and the ORF and another modification solely in the poly(A) tail. Such modification can be effected by performing first a transcription reaction to introduce one or more first types of modified nucleotide and then following up with a poly(A) polymerase reaction using a second type of modification-containing ATP.

It will be apparent to the skilled person that numerous modifications and combinations of modifications are possible in the context of the present invention. Further, it is also possible to include different labels or functional groups based on the presence of the alkyne- and/or azide-modifications on the mRNA molecule, but rather also by consecutive addition under click reaction conditions of different appropriately modified labels or functional molecules. Consequently, the present invention provides a vast number of options and a convenient modularity in order to adapt the modified mRNA to the intended use in an optimal manner.

Apart from including alkyne- and / or azide-modified nucleotides, the present invention generally also allows for other modifications in the nucleotides as far as such other modifications do not adversely affect mRNA production or the intended use of the resulting mRNA. As an example of such other modified nucleotide or nucleotide derivative that can be included in the mRNA, pseudouridine-5'-triphosphate (pseudo-UTP) can be considered. Pseudouridine (or 5'-ribosyluracil) was the first modified ribonucleoside that was discovered. It is the most abundant natural modified RNA base and is often designated as the "fifth nucleoside" in RNA. It can be found in structural RNAs, such as transfer, ribosomal and small nuclear RNA. Pseudouridine has been shown to enhance base stacking and translation. Further, pseudouridine-5'-triphosphate is able to impart advantageous mRNA characteristics such as increased nuclease stability and altered interaction of innate immune receptors with *in vitro* transcribed RNA. Pseudo-UTP and also further modified nucleotides, like *N*1-methylpseudouridine and 5-methylcytidine-5'-triphosphate, have shown innate immune suppression in culture and *in vivo* while simultaneously enhancing translation. Inclusion of these and other suitable nucleotides in alkyne- or azide- modified or in non-modified form is therefore a further option and preferred embodiment of the present invention.

As apparent from the above description of the modified mRNA of this invention, a multitude of different options exist to prepare or adapt an mRNA molecule to be beneficially applicable for various purposes. The invention is not restricted to a particular type of mRNA, which can rather be chosen in accordance with any intended use thereof, especially in the applications described in general or in more detail above in the background section as well as in the following. The mere introduction of the alkyne- or azide-modification conveys enhanced stability to an mRNA molecule which can be administered to deliver genetic information for applications like protein replacement therapy or to deliver mRNA for immunostimulatory purposes or as an mRNA-vaccine. Further modifying the mRNA via downstream click-coupling of respectively modified labels or functional molecules provides further possibilities for screening delivery of the modified mRNA and/or to target delivery of the mRNA to specific cells or tissues e.g. in a gene replacement therapy or to improve pharmacokinetics (e.g. slower renal clearance by adding PEG labels).

Within the context of the present invention, the modified mRNA of the present invention can be used together with substances which are required or preferably present for a certain application. For example, for *ex vivo* cell transfection but also for *in vivo* administration, substances which facilitate mRNA uptake by cells are preferably included with the mRNA. Lipid formulations as well as nanocarriers (e.g. as described by Moffett et al., mentioned supra) can preferably be included within the context of the present invention. Accordingly, a mixture of substances containing the modified mRNA and at least one other substance as mentioned above, or a kit of parts in which the modified mRNA and at least one other suitable substance are provided in different containers for subsequent combined use are further subjects of the present invention.

Another subject of the present invention is a process for producing the modified mRNA of the present invention. According to a first process, mRNA is transcribed *in vitro* from a DNA template in the presence of an RNA polymerase, usually T3, T7 or SP6 RNA polymerase, and a nucleotide mixture containing at least the four standard types of nucleotides (ATP, CTP, GTP, UTP) required for mRNA transcription and optionally naturally occurring modified nucleotides, like e.g. *N*1-methylpseudouridine triphosphate, or even suitable artificial nucleotides. In addition, to improve the translation efficiency it is important to generate a 5'-cap structure, e.g. 7-methylguanylate for eukaryotes. At least a part of at least one of the standard nucleotides, naturally occurring modified nucleotide analogue or suitable artificial nucleotide analogues is modified to contain an alkyne-or azide-modification at the nucleotide.

Depending on which type of nucleotide is used for the process, the modification will be effected in the UTRs and the ORF only (for modified CTP, GTP or UTP, or their analogues) or in all of the UTRs, the ORF and the poly(A) tail (for modified ATP alone or in combination with one or more of modified CTP, GTP or UTP, or their analogues).

As an alternative to *in vitro* transcription, also a fermentation process in prokaryotic or eukaryotic systems, for producing the mRNA of the invention is included in the context of the present invention. For this purpose, a DNA template, preferably a plasmid containing the DNA of interest, is introduced into the microorganisms and respective nucleosides or nucleotide prodrugs (to allow sufficient cellular uptake) as described above are included in the culture medium. Fermentative mRNA production is known to the skilled person, cf. e.g. Hungaro et al. (J Food Sci Technol. 2013 Oct; 50(5): 958-964).

A second process within the context of the present invention allows for modification of the poly(A) tail region only, by first providing an mRNA of interest and adding modified alkyne-or azide-modified ATP (or analog) in a poly(A) polymerase addition reaction.

While the first and the second process described above can be used to provide modified mRNA of the present invention, it is also possible to use a combination of both processes to include modified alkyne and/or azide-modified nucleotides with the UTRs and the ORF or the UTRs, the ORF and the poly(A) tail during the mRNA transcription step. By additionally performing the second process, i.e. a poly(A) polymerase addition reaction, a further extension of the poly(A) tail can be achieved, wherein ATP is at least partly included in an alkyne- or azide-modified form which optionally is different from the modification that is introduced by the first process.

The conditions and methods to perform *in vitro* mRNA transcription (IVT) as well as a poly(A) polymerase addition reaction are well known to the skilled person (e.g. Cao, G.J et al, N. Proc. Natl. Acad. Sci. USA. 1992, 89, 10380-10384 and Krieg, P. A. et al,. Nucl. Acids Res. 1984, 12, 7057-7070)

Such conditions and methods are not particularly critical as long as a satisfactory yield of modified mRNA is obtained. In this context, also the kind of DNA template used within the first described process is not particularly critical. Usually, DNA to be transcribed is included in a suitable plasmid, however it can also be used in linear form. Additionally, DNA template usually contains a promoter sequence, especially a T3, T7 or SP6 promoter sequence.

During the process of producing the modified mRNA of the present invention, the obtained mRNA is preferably capped using well-known methods (Muthukrishnan, S., et al, Nature 1975, 255, 33-37). Required reactants for the capping are commercially available, for example A.R.C.A. (P1-(5'-(3'-O-methyl)-7-methyl-guanosyl) P3-(5'-guanosyl)) triphosphate, a cap analog) (Peng, Z.-H. et al, Org. Lett. 2002, 4(2), 161-164). Preferably, as an alkyne-modified nucleotide, ethynyl-modified nucleotides, most preferably 5-ethynyl UTP or 7-ethynyl-7-deaza ATP, are included in the process. As an azide-modified nucleotide, preferably 5-(3-azidopropyl) UTP or 8-azido ATP is used.

Within the context of the present invention, it is preferred to perform the transcription process using T7 RNA polymerase and to provide the DNA template in a suitable vector for efficient template production using microorganisms and subsequent *in vitro* transcription after linearization of the vector. The process can be performed using only one type of modified nucleotide or including one or more nucleotides comprising desired alkyne- or azide-modification. Within the context of the present invention, it is preferred to include one or two types of equally modified nucleotide, most preferably alkyne- or azide-modified uracil or adenine. As far as the alkyne-modification is concerned, it is most preferable to include an ethynyl group which, due to its size, is least prone to negatively affect the transcription reaction.

In another preferred embodiment of the invention, two differently modified nucleotides are included with the nucleotide mixture during transcription. Such a process results in a modified mRNA molecule which contains an alkyne- as well as an azide-modification.

No particular restrictions have been observed concerning the amount of modified nucleotides to be included during transcription. Theoretically, all nucleotides employed in the *in vitro* transcription can be modified to contain alkyne- or azide-modified nucleobases. It is, however, preferred to use one or two types of modified nucleotides and also to include such nucleotides in modified as well as in non-modified form. Depending on the desired modification rate, it is preferred to include modified versus non-modified forms of the various nucleotides in a ratio of 1:100 to 10:1, preferably 1:10 to 10:1 and further preferably 1:4 to 4:1 or 1:2 to 2:1. Most preferably, only one type of modified nucleotide is employed which can be present in modified form only, or in combination with the non-modified form in the above-mentioned ratios. Preferably, a 1:1, 1:2 or 1:10 combination of modified to non-modified nucleotide is provided.

It is further possible and can be desirable to include differently modified natural nucleotides, e.g. pseudouridine or *N*1-methyl-pseudouridine and/or artificial nucleotides or nucleotide derivatives to improve mRNA stability and enhance translation of the produced mRNA. More information with regard to differently modified nucleotides and their incorporation into mRNA during *in vitro* transcription can be derived from Svitkin, Y.V. et al., Nucleic Acids Research 2017, Vol. 45, No. 10, 6023-6036.

Modified mRNA of the invention which is produced by *in vitro* mRNA transcription or by (poly)A polymerase addition reaction on an existing mRNA of interest and comprises at least one of an alkyne- or azide-modification can further be reacted via a click reaction to incorporate other molecules of interest, especially labels and/or functional molecules as already explained above. For example detectable labels like e.g. fluorescent or colored molecules or non-colored molecules as mentioned earlier can be introduced. As a consequence, e.g. delivery of the generated mRNA can be monitored using fluorescent microscopy or other detection methods, especially in cell culture experiments. It surprisingly has been observed that even relatively big modifications of the bases within the ORF are accepted during translation of mRNA at the ribosome. For example, Cyanine 5 modified eGFP (enhanced green fluorescent protein) mRNA is commercially available (TRILINK biotechnologies, product code LL7701) that contains Cyanine 5 (Cy5) modified uridines. Such mRNA is readily translated to a functional protein in cell culture.

Selective modification of solely the poly(A) tail region can be achieved as described above, when poly(A) polymerase adds azide- or alkyne-modified ATP or ATP derivatives to the mRNA. Subsequent click labeling of this modified poly(A) tail has only minor effects on translation (as the sequence is not translated) and can be used, e.g. for tissue-specific ligands that mediate targeted uptake of the mRNA or to increase mRNA stability against nuclease degradation, as explained above. Especially in cases in which it is desired to attach very large molecules or molecules that due to other reasons impair translation, coupling via the poly(A) tail can be a preferred or even a mandatory approach.

The click reaction is well known to the skilled person and it is generally referred to Sharpless et al. and Meldal et al., mentioned supra. The overall conditions for the click reaction are described in these documents and it is further referred to disclosure in Himo F. et al., J. Am. Chem. Soc., 2005, 127, 210-216, which relates to the preferred copper-catalyzed azide-alkyne cycloaddition (CuAAC). It is also referred to EP 2 416 878 B1 with regard to the conditions and reactants for the click reaction as well as to EP 17 194 093, wherein a preferred method for coupling a first molecule to a second molecule in a click ligation reaction is described. In this context, the copper-catalyzed click reaction is performed in the presence of divalent metal cations in the reaction mixture, preferably in the presence of Mg²⁺.

While the above-mentioned documents describe click reactions in the context of ligating DNA molecules, in general, the same conditions can be applied within the context of the present invention. Thus, the click reaction is preferably carried out in the presence of a heterogeneous Cu (I) catalyst. Further, it is preferred to include a Cu (I) stabilizing ligand and/or organic solvents, especially DMSO to improve the efficiency of the click reaction, and/or divalent cations.

In a further preferred embodiment, the click reaction is performed as a strain-promoted azide alkyne cycloaddition reaction (SPAAC) as described earlier with regard to the modified mRNA of the invention. The exact conditions of a CuAAC or a SPAAC reaction can be adapted to the individual circumstances as long as the basic requirements that are known to the skilled person are observed. As mentioned above, SPAAC can also be performed inside of cells. Introducing an alkyne- or azide-modified label into such cells can be useful in order to, after transfection of a modified mRNA into cells, monitor e.g. the location of the mRNA in the cell.

The present invention allows to produce in a modular and highly efficient manner modified mRNA molecules which contain modifications which impact a stabilizing effect on the mRNA. The modifications are also useful as anchor molecules to which other substances and molecules can be linked via a click reaction. Such click reaction is performed downstream and separately from the transcription reaction which is a tremendous advantage, especially where large and bulky molecules of interest are to be ligated to the mRNA, which would completely disrupt the transcription reaction.

Within the context of the present invention, it can be sufficient that only a small set of alkyne- and / or azide-modified nucleotides are incorporated during *in vitro* mRNA production to allow synthesis of a whole range of densely modified mRNAs. This allows for a fast preparation and screening of many modifications. Moreover, the mRNAs of the present invention and processes for producing same permit to incorporate functional groups that are not readily or not at all accepted by the RNA polymerases during mRNA production, but are easily attached via click reaction after transcription. Incorporation of such functional groups cannot be effected by conventional methods.

Thus, the inventive mRNAs and the processes for their production for the first time provide an easy and reliable method to produce stabilized and customarily modified mRNAs which can be labelled to follow-up on their uptake for example in *ex vivo* cell transfection and can also be modified to provide improved cell- or tissue-specific targeting for specific uses in therapy or vaccine preparation.

One preferred application of the mRNAs of the present invention lies in transfection of target cells *ex vivo.* As mentioned in the background section, mRNA formulations which are applied systemically and especially intravenously are taken up mainly by liver cells, whereas very often cells of the immune system are the preferred target in order to evoke an immune stimulatory effect or when mRNA is used for direct vaccination. In case that it is desired to incorporate the modified mRNA of the invention into specific cell types, such cells can be isolated from a patient, especially from a patient's blood, and mRNA transfection can be performed *ex vivo.*

A further subject of the present invention, therefore, is a cell preparation and especially a preparation of cells of the immune system, which includes a modified mRNA of the present invention and is obtained by *ex vivo* transfection of cells.
In principle, the modified mRNA of the present invention can be used to transfect any kind of cell, human, animal or also plant cells. In one aspect of the invention, the cells of the cell preparation are of animal or human origin.

This aspect of the invention relates to inter alia adoptive cell transfer (ACT) and its manifold applications and uses which have been developed within the last decades. Autologous as well as non-autologous cells can be treated in order to for example improve immune functionality and other characteristics. Preferably, cells of the immune system are obtained from a patient and engineered to produce autologous immune cell which have been proven valuable in treating various diseases including cancer, e.g. B-cell lymphoma. The CAR-T cell based therapy is one such approach in which T-cells are genetically engineered to produce chimeric antibody receptors on their surface which recognize and attach to a specific protein or antigen on tumor cells.

The cell preparations of the present invention can be used in the same context. Depending on the modified mRNA which is introduced into cells, ensuing expression of a protein can provide for a multitude of effects of such cells after (re-)application to a patient. The present cell preparations, accordingly, are not restricted to a small number of applications but rather can be considered a vehicle for expression of mRNA, *in vivo* after (re)application of cells which then produce a protein of interest and/or exert a certain effect (e.g. immune stimulating or tolerogenic) in the patient due to expression of the protein.

Methods for cell transfection are known to the skilled person and can be adapted to the particular cell type of interest. As an example for such process, it is referred to Moffett et *al*., mentioned supra. In the context of *ex vivo* transfection it is especially preferred to use an mRNA of the invention which is modified via click reaction to contain a cell-specific targeting group which facilitates uptake of the mRNA into the cell without transfection agents, since immune-cells are especially damageable by some of these transfection agent components.

In addition to *ex vivo* transfection of cells and administering such transfected cells to a patient, the modified mRNA of the present invention can also be applied directly to a patient. Both cases are considered a therapeutic (or also prophylactic) treatment. A further subject-matter of the present invention therefore is a pharmaceutical composition which comprises a modified mRNA or a cell preparation of the present invention as an active agent. As already mentioned, mRNA based therapeutics have recently become important research subjects. A large number of applications for mRNA as therapeutic agents has been described (e.g. Sahin et al, Schlake et al. and Kranz et al, all mentioned supra) and the modified mRNA of the present invention can not only be used in all such applications but can even provide for advantages and improvements thereto. Based on the enhanced stability of the modified mRNA and further based on an optionally present functional group, various problems can be solved. An enhanced stability accounts for e.g. a prolonged translation into protein compared to a non-modified mRNA. Further, the presence of a tissue- or cell-targeting group allows for targeted administration and high specificity of a therapeutic or immunogenic treatment.

Among suitable applications of the modified mRNAs, the cell preparations and the pharmaceutical compositions containing such mRNA or cell preparation are gene or protein replacement therapy, targeted transient gene delivery and genome engineering/gene editing (e.g. mRNA coding for a targeting endonuclease and a guide RNA like in the CRISPR/Cas9 system or similar), infectious disease vaccination, cancer immunotherapy, as well as cell-specific gene expression for a treatment of inherited diseases.

Gene replacement therapy can be considered for the treatment of a large number of diseases. For many diseases for which a deficiency or malfunction in a protein or enzyme is a leading cause or consequence, administration of the required active protein to the patient is essential to avoid immediate or consequential damage. However, continuous administration of proteins can cause intolerance or other negative side effects.

Furthermore, in order to provide sufficient amounts of a certain protein to a patient, high concentrations of such proteins need to be administered, sometimes as high as 100 mg/ml and up to 20 g of the protein per day and patient. As a further problem in protein replacement therapy, it can also be difficult to administer the protein into cells. On the other hand, it could be shown that for some mRNAs it is sufficient to provide 50 to 100 µg per dose to achieve a sufficiently high intracellular protein level in patients. Thus, the present invention and especially the possibility to target specific cells or tissues, provides a convenient solution to problems encountered with current protein replacement therapy approaches. By using modified mRNAs of the present invention in protein replacement therapy, it is for example in many cases sufficient to inject the mRNA whereas current protein replacement therapies require infusions which usually are time consuming and physically demanding for the patient.

Examples of diseases requiring protein replacement or at least constant protein supplementation include protein deficiency diseases, many metabolic diseases like type I diabetes, and also inherited disorders, especially lysosomal storage diseases like Morbus Gaucher or Morbus Hunter.

In the context of gene replacement therapy, including the modified mRNA into cells *ex vivo* as well as *in vivo* can be considered. Especially if a cell- or tissue-specific targeting-group is included with the mRNA of the invention, even *in vivo* insertion of the modified mRNA into target cells is expected to be highly efficient. The present invention allows for an endogenous translation of mRNA into protein, thus, avoiding adverse effects as mentioned above. Nevertheless, also *ex vivo* insertion of the mRNA into target cells and (re)administration of such target cells into patients is a further option within the context of the present invention, as mentioned above.

The pharmaceutical composition according to the invention can also be applied as mRNA vaccine. Vaccination is effected based on *in situ* protein expression to induce an immune response. Since any protein can be expressed from the modified mRNA of the present invention, the present pharmaceutical compositions can offer maximum flexibility as regards the desired immune response. Using the modified mRNA also provides a very fast immunization alternative compared to conventional methods for which it is necessary to produce various protein constituents or even inactivated viral particles. Conventional methods usually require performing different production processes whereas using the present invention, various mRNAs encoding different proteins or protein parts relating to the infectious agent can be produced in the same preparation process. Immunization by mRNA vaccination can even be achieved by single vaccinations and using only low mRNA doses. As opposed to DNA vaccines, RNA vaccines do not need to cross the nuclear envelope, but it rather is sufficient for them to reach the cell cytoplasm by crossing the plasma membrane. Further information regarding the development of mRNA vaccines is disclosed e.g. in Schlake *et al.,* mentioned supra, and is applicable also in the context of the present invention. The pharmaceutical composition including the modified mRNA of the invention can be employed as prophylactic as well as therapeutic vaccines. The vaccines can be directed against any kind of pathogens, e.g. viruses like Zika virus which recently has become a major focus of attention (Pardi et al, mentioned supra).

In addition to a vaccination against exogenous pathogens, the pharmaceutical compositions of the present invention can also be used as anti-tumor vaccines or to stimulate the immune system within the context of cancer immunotherapy. Especially systemic RNA delivery to dendritic cells or macrophages offers the possibility to exploit antiviral defense mechanisms for cancer immunotherapy as described by Kranz *et al.,* mentioned supra. Targeting e.g. macrophages or dendritic cells with an mRNA expressing a protein specific to or within the context of a certain type of cancer leads to presentation of parts of such protein by MHC molecules and elicits a potent and specific immune response. Accordingly, the modified mRNA of the present invention can also be used in the context of antigen-encoding mRNA pharmacology.

Targeted gene editing using specific endonucleases and guide RNAs is a further application of the modified mRNA or pharmaceutical composition of the present invention.

The recently developed, ground-breaking CRISPR/Cas9 technology enables specific gene editing, allows to introduce, delete or silence genes and is even able to exchange nucleotides within a gene. The method described by Charpentier and Doudna involves Cas-proteins which are ribonucleoproteins and endonucleases which bind to specific chemically synthesized CRISPR RNA (crRNA) sequences and cut DNA in the vicinity of such RNA sequences. In order to direct the endonuclease activity to the desired target DNA sequence, a so called guide RNA is used which is complementary to the target DNA sequence. The guide RNA can take two forms, either a complex of a long, chemically synthesized trans-activating CRISPR RNA (tracrRNA) plus the crRNA, or a synthetic or expressed single guide RNA (sgRNA) that consist of both the crRNA and tracrRNA as a single construct. The modified mRNA of the present invention can be used in this context to encode one or both of the endonuclease and the guide RNA, preferably as a sgRNA which is complementary to a specific DNA sequence of interest.

The major advantage of the transient expression of the gene editing endonuclease and guide RNA from mRNA compared to the current technology, is the reduced risk of nonspecific gene editing, since the gene expression of the genetic tool from mRNA is limited to a short period of time (a few days) and not constantly expressed from an integrated genome element.

Gene editing applications have high importance and can be applied in the context of a huge variety of therapeutic applications, e.g. gene replacement therapy, cancer therapy and treatment of inherited diseases. Use of the modified mRNA of the invention in the context of all such applications is included within the scope of the present invention.

Also for other potential therapeutic applications described for mRNAs in the prior art or which are developed in the future, use of respective mRNAs including the modifications as described herein is of advantage due to their improved stability. Further, including a tissue- or cell-specific targeting molecule to the mRNA via the downstream click reaction, allows for a more specific and accordingly more efficient use in therapy. Especially any desired protein expression or also any immunization reaction can be precisely targeted to the location in a patient where the therapeutic or immunizing effect is required.

A preferred embodiment of the pharmaceutical composition of the present invention includes the modified mRNA together with a pharmaceutically acceptable carrier, excipient and/or adjuvant. In a further preferred embodiment, the pharmaceutical composition comprises complexing agents, which protect the mRNA further from degradation. The complexing agent may further improve and enhance uptake by cells and concurrent translation into protein. As complexing agents, lipids or polymers can be included in the pharmaceutical composition. In a further preferred embodiment, the pharmaceutical composition can contain the modified mRNA encapsulated in liposomes.

In a further preferred embodiment, the pharmaceutical composition contains cationic lipids. Agents that further improve the delivery of nucleic acids to the cytosols can also be preferably included in the pharmaceutical compositions of the present invention. Such agents can be tailored to the specific route of delivery. In summary, the pharmaceutical compositions of the present invention, while including the inventive modified mRNA as active agent, can include any further substances for improving further the stability of the active substance, enhancing delivery to the cytoplasm of target cells and providing other complementary or synergistic effect.

Within the context of the present invention, a pharmaceutical composition is included which as an active agent contains a cell preparation, especially a preparation of cells of the immune system, which is obtained by *ex vivo* transfection of cells with a modified mRNA of the present invention. The transfected cells can be returned to the patients in order to benefit from the effects of the modified RNA included in the cells. Also for this preferred embodiment of a pharmaceutical composition of the invention, pharmaceutically acceptable adjuvants or excipients or carriers can be included as outlined before.

A further subject of the present invention is a diagnostic composition containing a modified mRNA of the present invention or a cell transformed with a modified mRNA of the present invention for *in vitro* or *in vivo* screening for the presence, delivery and/or distribution of the inventive mRNA in cells, tissues or organs. For such purpose, preferably the modified mRNA already includes a detectable label which was introduced via a click reaction. Such label preferably is a fluorophore or a radionuclide, preferably a positron emitting radionuclide. Detecting and possibly also quantifying the detectable label allows to observe and detect delivery of the modified mRNA to cells, tissues or organs, the distribution therein or to monitor the re-administration of cells into a patient. Accordingly, a diagnostic composition of the present invention contains a modified mRNA of the invention, preferably an mRNA containing at least one detectable label.

In a further aspect of the present invention and as mentioned earlier, also plant cells can be transfected using the modified mRNA of the present invention. Such transfected plant cells are also encompassed within the scope of the present invention. The modified mRNA can be included e.g. in order to introduce genetic information, especially for transient expression of certain proteins in such plant cells, or for analytic or diagnostic purposes e.g. as labeled probes. Conferring disease or pest resistance or tolerance are only some examples of possible applications and beneficial effects of introducing inventive mRNAs into plant cells or plants. The use of a modified mRNA of the present invention for transfection of plant cells or plants, accordingly, is also a subject of the present invention.

Still a further subject of the present invention is a kit for preparing a modified mRNA of the present invention. Such kit contains the various substances required for preparing the modified mRNA, i.e. an RNA polymerase and/or poly(A) polymerase, alkyne- or azide-modified nucleotides as well as unmodified nucleotides and optionally further buffer substances and solvents. In preferred embodiments, also alkyne- and/or azide-modified detectable labels or functional molecules as well as substances required for performing the click reaction between the modified mRNA and the labels or functional molecules are included. The substances can be present in separate containers or can be combined as far as no adverse reaction occurs between such combined substances. As regards the various substances to be included in such kit of parts, it is referred to the above description regarding the modified mRNA of the invention and the processes for preparing such modified mRNA. The kits according to the present invention can also contain further substances facilitating the delivery of the modified mRNA of the invention to cells *ex vivo* or *in vivo.*

In preferred embodiments, the kit contains a modified mRNA containing a detectable label or a functional molecule introduced via click reaction, or it contains the modified mRNA and the alkyne- or azide-modified label or molecule and eventually the click reagents in separate containers. It will be apparent to the skilled person that many different combinations of substances can be included in kits which facilitate the preparation or the use of the modified mRNA of the invention. All embodiments and variations thereof described above in the context of the present invention are also applicable for the kits described here. All suitable combinations of substances are included for the purposes of the present invention.

All information disclosed above with regard to one subject of the present invention is considered to equally apply in the context of other subjects for which this information, even if not explicitly repeated, has recognizable relevance within the context of the invention.

### Brief Description of the Figures

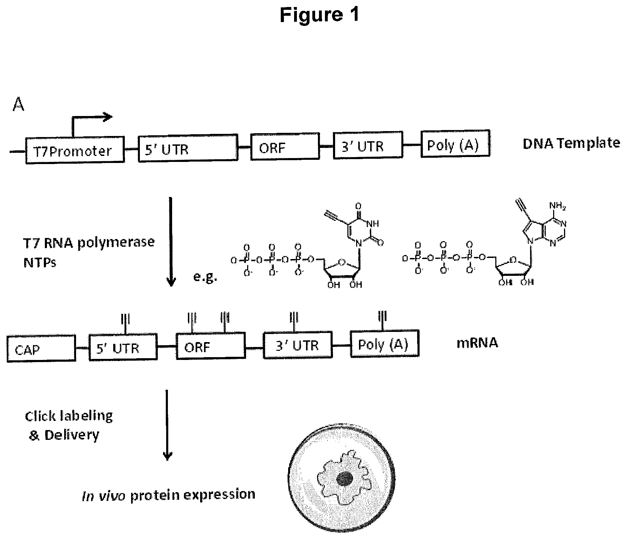

Figure 1 shows a general scheme of modified mRNA production and application. Using e.g. 5-ethynyl UTP (EUTP) it is possible to insert alkyne groups available for click reaction with the 5'-UTR, 3'-UTR and the ORF. Selective labelling of the poly(A) tail is possible using e.g. 7-ethynyl 7-deaza ATP and a poly(A) polymerase.
Figure 2 shows the general schematic production of alkyne-modified mRNA using e.g. T7 RNA polymerase and a nucleotide mixture including EUTP (structural formula).
Figure 3 shows the results of transfection of non-alkyne (A), alkyne (B) and dye (C) modified mRNA coding for eGFP into HeLa cells.
Figure 4 shows the general schematic production of alkyne-modified mRNA using e.g. poly(A) polymerase and the alkyne modified nucleotide EATP (structural formula).
Figure 5 shows the results of transfection of Eterneon-red 645 modified mRNA coding for eGFP into HeLa cells.
Figure 6 shows a map and the complete sequence (from T7 promoter to poly(A) end) of the plasmid used in linearized form as DNA template during the T7 RNA polymerase reaction in the Examples. The sequence is also referred to as SEQ ID NO: 2.
Figure 7 shows the result of experiments to prove internalization of EATP with the poly(A) tail of an mRNA in Example 3.

The following examples further illustrate the invention:

### Examples

### Example 1:

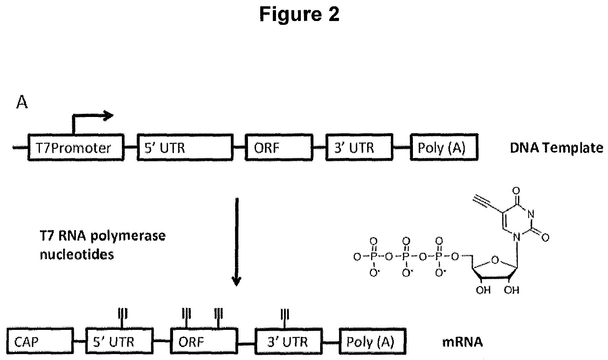

Alkyne-modified mRNA coding for the enhanced green fluorescent protein (eGFP) was produced by *in vitro* transcription (IVT) from a DNA template using T7 RNA polymerase and nucleotide mixtures. Here 5-ethynyl-uridine-5'-triphosphate (EUTP) was included in the nucleotide mixture to generate an alkyne-modified mRNA according to Figure 2 for subsequent transfection into Henrietta Lacks' immortal cells (HeLa cells).The generated mRNA contains a 5'-cap, untranslated regions (UTR), the protein coding part (open reading frame, ORF) and a poly(A) tail.

### mRNA production

In a 50 µL reaction volume 20 units of T7 RNA polymerase, 1 µg of template DNA and several nucleotides were combined in transcription buffer (40 mM Tris-HCl, pH 7.9, 6 mM MgCl₂, 4 mM spermidine, 10 mM DTT).
A) Final nucleotide concentrations for non-alkyne modified mRNA production were:
   1.0 mM GTP, 4.0 mM A.R.C.A. (P1-(5'-(3'-O-methyl)-7-methyl-guanosyl) P3-(5'-(guanosyl))triphosphate, Cap Analog), 1.25 mM CTP, 1.25 mM UTP, 1.25 mM ψUTP (pseudouridine triphosphate), 1.5 mM ATP.
B) Final nucleotide concentrations for alkyne modified mRNA production were:
   1.0 mM GTP, 4.0 mM A.R.C.A. (P1-(5'-(3'-O-methyl)-7-methyl-guanosyl) P3-(5'-(guanosyl))triphosphate, Cap Analog), 1.25 mM CTP, 1.25 mM EUTP (5-ethynyluridine triphosphate), 1.25 mM ψUTP (pseudouridine triphosphate), 1.5 mM ATP.
C) Final nucleotide concentrations for alkyne modified mRNA production and subsequent click labeling were:
   1.0 mM GTP, 4.0 mM A.R.C.A. (P1-(5'-(3'-O-methyl)-7-methyl-guanosyl) P3-(5'-(guanosyl))triphosphate, Cap Analog), 1.25 mM CTP, 0.625 mM EUTP (5-ethynyluridine triphosphate), 0.625 mM ψUTP (pseudouridine triphosphate), 0.625 mM UTP, 1.5 mM ATP.

The mixture was incubated for 2 hours at 37 °C and then 2 units of DNAse I were added and incubated for 15 minutes at 37 °C. The mRNA was purified by a spin column method according to manufacturers' instruction for PCR products (PCR purification kit from Qiagen). This yielded 13.3 µg of mRNA for A, 12.3 µg B and 14.3 µg for C, which was directly used for transfection when no click labeling was needed (A and B). When click labeling was performed (C), 2 µg of RNA, 1 nmol Eterneon Red 645 Azide (baseclick GmbH), a single reactor pellet and 0.7 µL 10x Activator² (baseclick GmbH, Oligo² Click Kit) were combined in a total reaction volume of 7 µL. The reaction mixture was incubated at 45 °C for 30 min and then cleaned using a spin column method according to manufacturers' instruction for PCR products (PCR purification kit from Qiagen).

For transfection a commercial kit (jetMESSANGER™ from POLYPLUS TRANSFECTION®) was used according to manufacturers' instructions using 0.5 µg of mRNA and 25,000 HeLa cells (CLS GMBH) reaching confluence. The cells were incubated at 37 °C for 24 hours before analysis under the fluorescent microscope, GFP filter: (470/22 excitation; 510/42 emission) and Cy5 filter (628/40 excitation; 692/40 emission) were used.

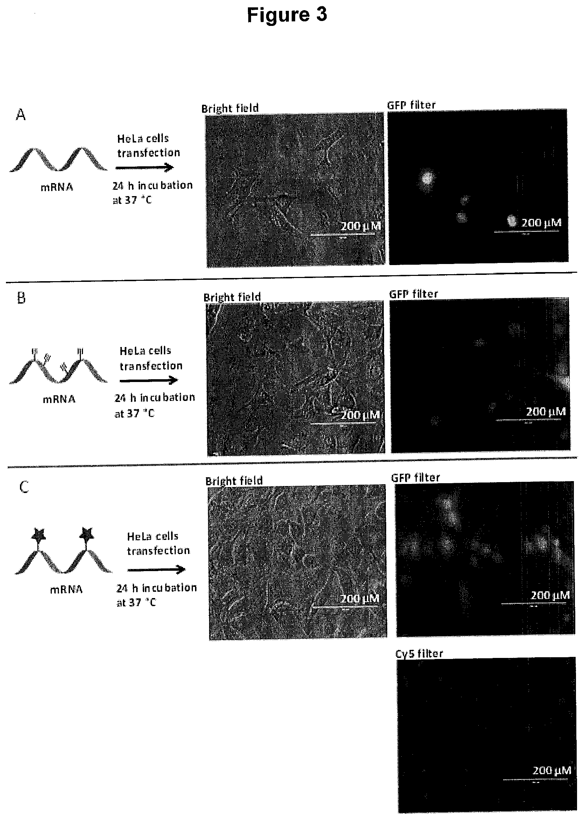

Figure 3 shows the results of transfection of non-alkyne (A), alkyne (B) and dye (C) modified mRNA coding for eGFP into HeLa cells. After 24 h incubation at 37 °C green fluorescence of the eGFP was observed (GFP filter). For the Eterneon Red labeled mRNA (C) the localization of the mRNA was observed using Cy5 filter settings.

In the bright field image cell morphology of healthy HeLa cells was observed (Figure 3, A-C), using the GFP filter protein expression of the eGFP was visible (exposure time 120 ms for A-B, 250 ms for C). For the click labeled mRNA (Figure 3, C) also the localization of the mRNA was observed using the Cy5 filter settings of the microscope.

### Supporting Information:

Structure of the modified nucleotides used during the T7 RNA polymerase reaction described above.

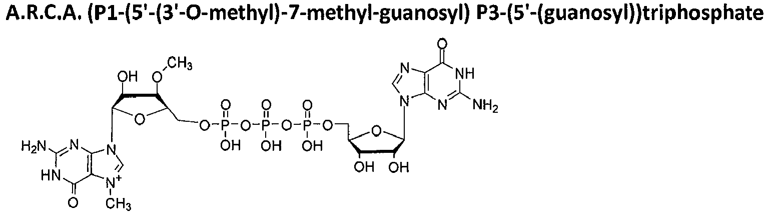

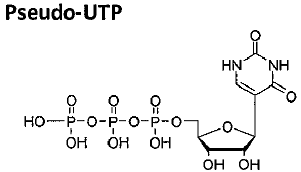

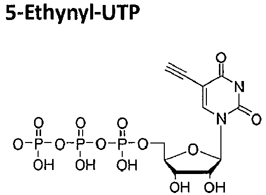

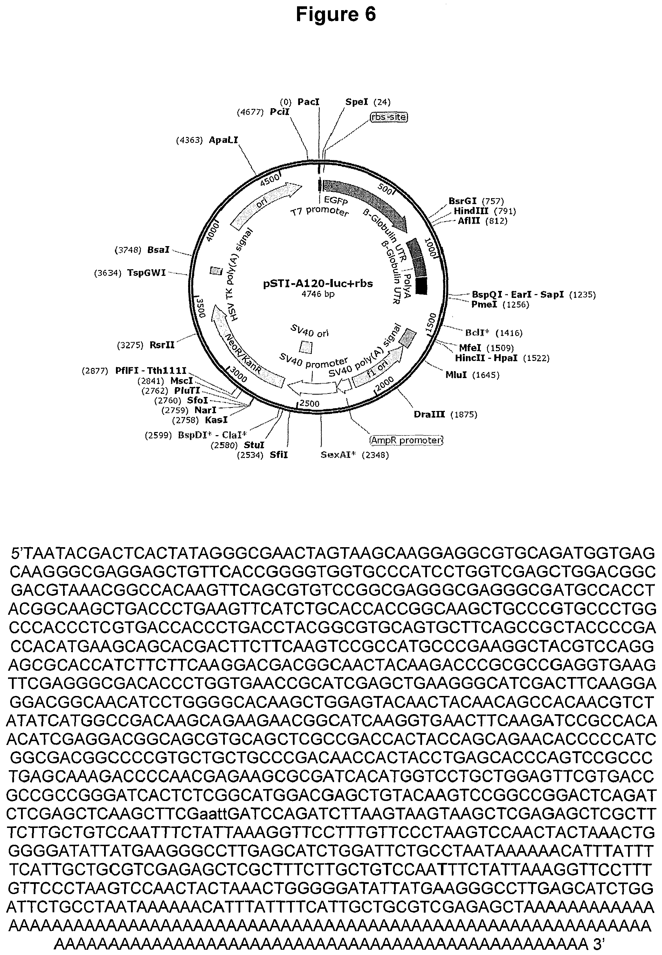

The map and complete sequence (from T7 promoter to poly(A) end) of the plasmid used in a linearized form as DNA template during the T7 RNA polymerase reaction described above is shown in Figure 6.

### Example 2:

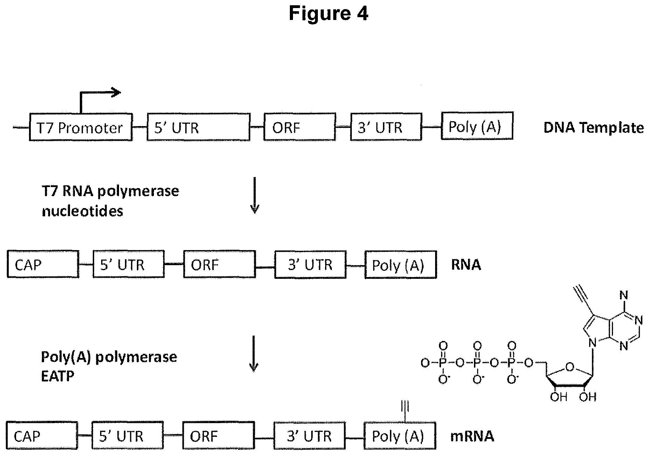

Alkyne-modified mRNA coding for the enhanced green fluorescent protein (eGFP) was produced by *in vitro* transcription (IVT) from a DNA template (Figure 6) using T7 RNA polymerase and nucleotide mixtures. Here 7-ethynyl-adenine-5'-triphosphate (EATP) was incorporated in the IVT mRNA after the T7 RNA polymerase reaction by poly(A) polymerase to generate an alkyne-modified mRNA according to Figure 4 for subsequent click labeling and transfection into Henrietta Lacks' immortal cells (HeLa cells).The generated mRNA contains a 5'-cap, untranslated regions (UTR), the protein coding part (open reading frame, ORF) and a poly(A) tail alkyne labeled.

### mRNA production

In a 50 µL reaction volume 20 units of T7 RNA polymerase, 1 µg of linearized template DNA and several nucleotides were combined in transcription buffer (40 mM Tris-HCl, pH 7.9, 6 mM MgCl₂, 4 mM spermidine, 10 mM DTT).

Final nucleotide concentrations for non-alkyne modified mRNA production were:
1.0 mM GTP, 4.0 mM A.R.C.A. (P1-(5'-(3'-O-methyl)-7-methyl-guanosyl) P3-(5'-(guanosyl))triphosphate, Cap Analog), 1.25 mM CTP, 1.25 mM UTP, 1.25 mM ψUTP (pseudouridine triphosphate), 1.5 mM ATP.

The mixture was incubated for 2 hours at 37 °C and then 2 units of DNAse I were added and incubated for 15 minutes at 37 °C. The mRNA was purified by a spin column method according to manufacturers' instruction for PCR products (PCR purification kit from Qiagen). This yielded 12.3 µg of mRNA which was directly used for poly(A) polymerase reaction with EATP.

In a 20 µL reaction volume 5 units of *E.coli* poly(A) polymerase, 4.2 µg of mRNA prepared before and a solution of 1 mM EATP were combined in reaction buffer (250 mM NaCl, 50 mM Tris-HCl, 10 mM MgCl₂, pH 7.9)

The mixture was incubated for 1 hour at 37 °C. The mRNA was purified by a spin column method according to manufacturers' instruction for PCR products (PCR purification kit from Qiagen). This yielded 4 µg of mRNA.

The click labeling was performed using 1.1 µg of RNA, 1 nmol Eterneon Red 645 Azide (baseclick GmbH), a single reactor pellet and 0.7 µL 10x Activator² (baseclick GmbH, Oligo² Click Kit) were combined in a total reaction volume of 7 µL. The reaction mixture was incubated at 45 °C for 30 min and then cleaned using a spin column method according to manufacturers' instruction for PCR products (PCR purification kit from Qiagen).

For transfection a commercial kit (jetMESSENGER™ from POLYPLUS TRANSFECTION®) was used according to manufacturers' instructions using 0.5 µg of mRNA and 25,000 HeLa cells (CLS GMBH) reaching confluence. The cells were incubated at 37 °C for 24 hours before analysis under the fluorescent microscope, GFP filter: (470/22 excitation; 510/42 emission) and Cy5 filter (628/40 excitation; 692/40 emission) were used.

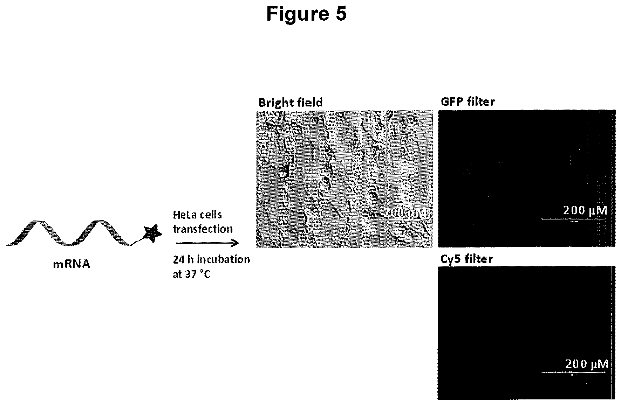

Figure 5 shows the results of transfection of Eterneon Red modified mRNA coding for eGFP into HeLa cells. After 24 h incubation at 37°C green fluorescence of the eGFP was observed (GFP filter). For the Eterneon Red labeled mRNA the localization of the mRNA was observed using Cy5 filter settings.

In the bright field image cell morphology of healthy HeLa cells was observed (Figure 5), using the GFP filter protein expression of the eGFP was visible (exposure time 120 ms). Localization of the mRNA labeled with Et-Red was observed using the Cy5 filter settings of the microscope.

### Example 3:

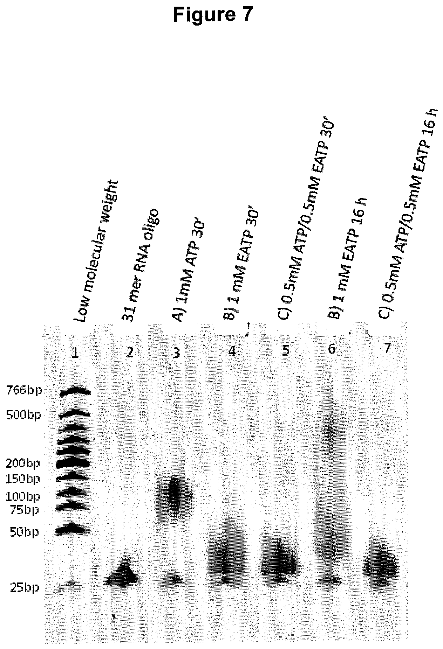

In order to prove incorporation of the EATP within the poly(A) tail a short RNA oligonucleotide (31 mer, CUAGUGCAGUACAUGUAAUCGACCAGAUCAA, SEQ ID NO: 1) was used as template for the poly(A) polymerase reaction using:
A) 1 mM ATP
B) 1 mM EATP;
C) 0.5 mM ATP and 0.5 mM EATP.
In a 20 µL reaction volume 5 units of *Escherichia coli* poly(A) polymerase, 2 µg of RNA (31 mer) and nucleotide (final concentration of A-C) were combined in reaction buffer (250 mM NaCl, 50 mM Tris-HCl, 10 mM MgCl₂, pH 7.9). The mixtures were incubated for 30 minutes at 37 °C or for 16 hours at 37 °C.
The results were analyzed by denaturing polyacrylamide gel electrophoresis (7 M urea, 1x TBE, 7% polyacrylamide gel, constant voltage 100 V, 1 h). Compared to the template RNA oligonucleotide (Figure 7, Lane 2) a band or smear at higher molecular weight appeared for all samples, which were incubated in the presence of the poly(A) polymerase using different nucleotides and incubation durations (Figure 7, Lane 3-7). This indicated successful incorporation of ATP or its alkyne analog EATP. Within 30 min incubation the incorporation of ATP (Figure 7, Lane 3) was more efficient compared to EATP (Figure 7, Lane 4) or a mixture of EATP and ATP (Figure 7, Lane 5). By extending the incubation time for the incorporation of EATP to 16 h, the length of the poly-EA-addition was increased (Figure 7, Lane 6) in comparison to 30 min incubation (Figure 7, Lane 4). Interestingly, for the nucleotide mixture containing ATP and EATP no change was observed after 16 h (Figure 7, Lane 7) compared to 30 min.

Figure 7 shows the ethidium bromide stained 7% denaturing polyacrylamide gel of different polyadenylation reactions as described above. In each lane 500 ng of RNA were loaded. Lane 1: low molecular weight DNA ladder (New England Biolabs), Lane 2: 31 mer RNA oligonucleotide template, Lane 3: polyadenylation reaction with 1 mM ATP for 30 min, Lane 4: like 3 but 1 mM EATP, Lane 5: like 3 but 0.5 mM EATP and 0.5 mM ATP, Lane 6: like 4 but 16 h incubation, Lane 7: like 5 but 16 h incubation.

## Claims

**1.** Modified messenger RNA (mRNA), comprising a 5'-cap structure, a 5'-untranslated region (5'-UTR), an open reading frame region (ORF), a 3'-untranslated region (3'-UTR) and a poly(A) tail region, **characterized in that** it contains at least one of an alkyne- or azide-modification in at least one nucleotide within at least one of the ORF, the 5'-UTR, the 3'-UTR and the poly(A) tail region.

**2.** Modified mRNA according to claim 1, **characterized in that** it contains modified nucleotides in
a) the ORF and the UTRs,
b) the ORF, the UTRs and the poly(A) tail, or
c) only the poly(A) tail.

**3.** Modified mRNA according to claims 1 or 2, wherein at least one of the four standard types of nucleotides (AMP, CMP, GMP, UMP) are partly or completely modified, preferably ethynyl- or azido-modified at uracil or adenine.

**4.** Modified mRNA according to anyone of claims 1 to 3, wherein at least one nucleotide is alkyne-modified and at least one nucleotide is azide-modified.

**5.** Modified mRNA according to any one of the preceding claims, wherein at least one of the four standard types of nucleotides is present in modified form compared to the non-modified form in a ratio of 1:100 to 10:1, preferably 1:10 to 1:10 or 1:1.

**6.** Modified mRNA according to any one of the preceding claims, **characterized in that** it contains otherwise modified natural or artificial nucleotides, preferably pseudouridine or *N*1-methylpseudouridine.

**7.** Modified mRNA according to any one of the preceding claims, wherein the modified mRNA contains one or more of a detectable label and a functional molecule introduced via a click reaction of the modified mRNA with a correspondingly modified alkyne- or azide-containing detectable label or functional molecule.

**8.** Modified mRNA according to claim 7, wherein the detectable label is a colored or fluorogenic molecule and/or the functional molecule is a tissue or cell specific targeting group or ligand, preferably a sugar moiety or a fatty acid moiety.

**9.** Process for the production of the modified mRNA according to any one of the preceding claims, wherein the process comprises *in vitro* transcribing mRNA from a DNA template or alternatively performing a fermentation process using microorganisms, in the presence of an RNA polymerase and a nucleotide mixture containing the four standard types of nucleotides required for mRNA transcription, in which nucleotide mixture at least a part of at least one of the four types of nucleotides is modified to contain an alkyne- or azide-modification.

**10.** Process for the production of a modified mRNA containing an alkyne- or an azide-modification at the poly(A) tail, wherein the process comprises performing a poly(A) polymerase addition reaction at the poly(A) tail on an mRNA in the presence of ATP, wherein ATP is at least partly alkyne- or azide-modified at the adenosine.

**11.** Process according to claims 9 or 10, further comprising adding a correspondingly alkyne- or azide-modified detectable label and/or functional molecule under conditions to perform a click reaction to produce a modified mRNA according to claims 7 or 8.

**12.** Cell, which is obtained by *ex vivo* transfection of a corresponding human, animal or plant parent cell with a modified mRNA according to anyone of claims 1 to 8.

**13.** Cell according to claim 12, wherein the cell is a cell of the human or animal immune system.

**14.** Pharmaceutical composition, comprising a modified mRNA according to anyone of claims 1 to 8 or a cell according to claims 12 or 13 as an active agent, optionally in combination with a pharmaceutically acceptable adjuvant or excipient and/or contained in pharmaceutically acceptable carrier.

**15.** Modified mRNA according to anyone of claims 1 to 8 or a pharmaceutical composition according to claim 14 for use in mRNA based therapeutic and/or prophylactic applications.

**16.** Modified mRNA according to anyone of claims 1 to 8 or pharmaceutical composition according to claim 14 for use according to claim 15, wherein the therapeutic and/or prophylactic application comprises targeted delivery in gene replacement therapy, targeted gene therapy in combination with specific endonucleases encoded by the mRNA (e.g. CRISPR/Cas9), in vaccination, in cancer therapy and for cell specific gene expression or gene editing for treatment of (inherited) diseases and genetic aberrations.

**16.** Modified mRNA according to anyone of claims 1 to 8 or pharmaceutical composition according to claim 14 for therapeutic and/or prophylactic use according to claims 15 or 16 in a human or an animal.

**17.** Use of a modified mRNA according to anyone of claims 1 to 8 for transfection of plants and plant cells.

**18.** A kit for production and/or delivery of a modified mRNA according to anyone of claims 1 to 8.

**19.** Diagnostic composition for the *in vitro* screening for the presence, delivery and/or distribution of the modified mRNA according to anyone of claims 1 to 8 in cells, tissue or organs, wherein the composition contains an mRNA including a fluorophore label or a radionuclide label.

**20.** Diagnostic composition for use in *in vivo* screening for the presence, delivery and/or distribution of the modified mRNA according to anyone claims 1 to 8 or for in vivo monitoring of the re-administration of a cell according to claims 12 or 13, wherein the composition contains a modified mRNA including a fluorophore label or a radionuclide label, or the cell is transfected by a modified mRNA including a fluorophore label or a radionuclide label.
